(19) Europäisches Patentamt
European Patent Office
Office européen des brevets

(11) Veröffentlichungsnummer: **0 015 005**
**B1**

# (12) EUROPÄISCHE PATENTSCHRIFT

(45) Veröffentlichungstag der Patentschrift: **25.04.84**

(21) Anmeldenummer: **80100876.4**

(22) Anmeldetag: **22.02.80**

(51) Int. Cl.³: **C 07 D 263/56,** C 07 D 277/64, C 07 D 235/12, A 01 N 43/76, A 01 N 43/78, A 01 N 43/52

## (54) Substituierte Phenoxialkancarbonsäurederivate, Verfahren zu ihrer Herstellung und ihre Verwendung als Herbizide.

(30) Priorität: **23.02.79 DE 2907089**

(43) Veröffentlichungstag der Anmeldung: **03.09.80 Patentblatt 80/18**

(45) Bekanntmachung des Hinweises auf die Patenterteilung: **25.04.84 Patentblatt 84/17**

(84) Benannte Vertragsstaaten: **AT BE CH DE FR GB IT NL**

(56) Entgegenhaltungen:
**DE - A - 1 670 630**
**DE - A - 2 036 421**
**DE - A - 2 640 730**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Koch, Manfred, Dr., Kreuzheck 2, D-6239 Eppstein/Taunus (DE)**
Erfinder: **Bieringer, Hermann, Dr., Eichenweg 26, D-6239 Eppstein/Taunus (DE)**

EP 0 015 005 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Substituierte Phenoxialkancarbonsäurederivate, Verfahren zu ihrer Herstellung und ihre Verwendung als Herbizide

Gegenstand der vorliegenden Erfindung sind neue heterocyclisch substituierte Phenoxialkancarbonsäurederivate der allgemeinen Formel I

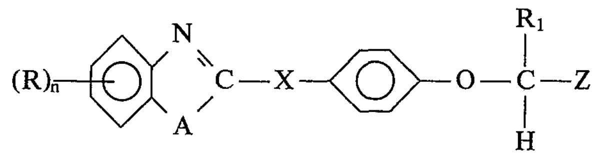

(I)

worin

R: Halogen, $CF_3$, $NO_2$, CN, $NH_2$, $(C_1-C_2)$-Alkyl, $(C_1-C_4)$-Alkoxi, $(C_1-C_4)$-Alkylthio oder Phenoxi, das gegebenenfalls ein- bis dreifach durch Halogen, $CF_3$, $(C_1-C_4)$-Alkyl, $NO_2$ oder die Gruppe $-NH-C(O)-R_2$ substituiert sein kann;
n: 0, 1, 2, 3 oder 4,
A: O, S, NH oder N-$(C_1-C_4)$-Alkyl,
X: $-CH_2-$ oder $-CH_2-CH_2-$,
$R_1$: H oder $(C_1-C_4)$-Alkyl
Z: eine Gruppe der Formel

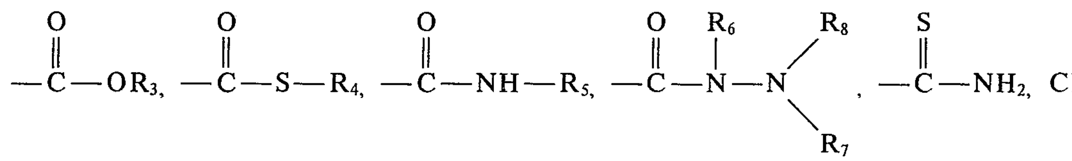

CN,

$-CH_2OH$, 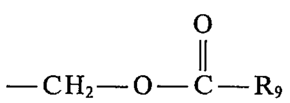 oder $-CH_2-O-SO_2-R_{10}$,

$R_3$: H, $(C_1-C_{12})$-Alkyl, das gegebenenfalls durch 1–6 Halogen, vorzugsweise F, Cl, Br und/oder durch OH, $(C_1-C_6)$-Alkoxi, $(C_1-C_4)$-Alkylthio, $(C_1-C_6)$-Alkoxi-$(C_2-C_6)$-alkoxi, Halogen-$(C_1-C_2)$-alkoxi, Methoxi-ethoxi-ethoxi, $(C_1-C_4)$-Alkylamino, Di-$(C_1-C_4)$-alkylamino, $(C_1-C_4)$-Alkoxicarbonyl, Phenyl, Oxiranyl und Phenoxi substituiert ist, wobei letzteres ebenfalls ein- bis zweifach durch Halogen oder $(C_1-C_4)$-Alkyl substituiert sein kann;
$(C_5-C_6)$-Cycloalkyl oder Halogen-$(C_5-C_6)$-alkyl;
$(C_3-C_6)$-Alkenyl, Halogen-$(C_3-C_6)$-alkenyl oder $(C_5-C_6)$-Cycloalkenyl;
$(C_3-C_4)$-Alkinyl, das gegebenenfalls ein- oder zweifach durch $(C_1-C_6)$-Alkenyl, Phenyl, Halogen bzw. $(C_1-C_2)$-Alkoxi substituiert ist;
Phenyl, das gegebenenfalls ein- bis dreifach durch $(C_1-C_4)$-Alkyl, $(C_1-C_4)$-Alkoxi, Halogen, $NO_2$ oder $CF_3$ substituiert ist; Furfuryl, Tetrahydrofurfuryl oder
ein Kationäquivalent einer organischen oder anorganischen Base ist;
$R_4$: $(C_1-C_6)$-Alkyl, das gegebenenfalls durch $(C_1-C_4)$-Alkoxi, Halogen oder Phenyl substituiert ist, wobei letzteres ebenfalls ein- bis dreifach durch $(C_1-C_4)$-Alkyl und Halogen substituiert sein kann;
$(C_3-C_6)$-Alkenyl oder Phenyl, das gegebenenfalls ein- bis dreifach durch $(C_1-C_4)$-Alkyl und/oder Halogen substituiert ist,
$R_5$: H, $(C_1-C_6)$-Alkyl, Hydroxy-$(C_1-C_6)$-Alkyl oder $(C_5-C_6)$-Cycloalkyl,
$R_6$: H oder $CH_3$,
$R_7$: H, $CH_3$ oder $C_2H_5$,
$R_8$: H, $CH_3$, $C_2H_5$ oder Phenyl,
$R_9$: $(C_1-C_6)$-Alkyl, das gegebenenfalls ein- bis dreifach durch Halogen substituiert ist,
$C_3$-, $C_5$- oder $C_6$-Cycloalkyl, $(C_3-C_6)$-Alkenyl, Phenyl, $(C_1-C_4)$-Alkylphenyl, $(C_1-C_4)$-Alkoxiphenyl, Halogenphenyl, Trifluormethylphenyl oder Nitrophenyl sowie
$R_{10}$: $(C_1-C_4)$-Alkyl oder Phenyl, das gegebenenfalls ein- bis dreifach durch Halogen, $CF_3$, $NO_2$ oder $(C_1-C_4)$-Alkyl substituiert ist, bedeuten.

Die Verbindungen der Formel I sind wertvolle Herbizide mit besonderer Wirkung gegen Schadgräser.

Aus GB-PS 1 157 558 sind bereits Verbindungen der Formel I bekannt, in denen Z für verschiedene (hier nicht beanspruchte) Carbonamido- und Carbanilidoreste steht. Die Verbindungen dieses Patents besitzen gefäßerweiternde Eigenschaften; eine herbizide Wirkung wird von ihnen nicht berichtet. Versuche haben jedoch gezeigt, daß auch diesen Verbindungen eine gewisse herbizide Wirksamkeit zukommt. Ferner sind aus DE-A-2 640 730 Verbindungen bekannt, die in der Position X anstelle einer

Alkylengruppe O oder S enthalten. Gegenüber diesen zeichnen sich die erfindungsgemäßen Verbindungen durch eine gute selektive Wirksamkeit gegen monokotyle Schadpflanzen aus.

Die in den Resten $R_1$—$R_5$; $R_9$ und $R_{10}$ aufgeführten Alkyl-, Alkenyl- oder Alkinylreste können sowohl geradkettig als auch verzweigt sein.

Bevorzugte Verbindungen der allgemeinen Formel I sind solche, in denen

R: Halogen, ($C_1$—$C_2$)-Alkyl
n: 0, 1 oder 2
A: O, S, N-($C_1$—$C_4$)-Alkyl,
X: $-CH_2-$ oder $-CH_2-CH_2-$
$R_1$: H oder $CH_3$
Z: eine Gruppe der Formel

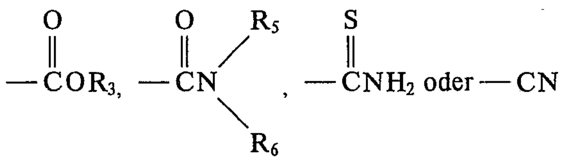

$R_3$: H, ($C_1$—$C_8$)-Alkyl, das gegebenenfalls durch Halogen, ($C_1$—$C_6$)-Alkoxi substituiert ist; oder ein Kationenäquivalent einer organischen oder anorganischen Base ist,
$R_5$: H oder ($C_1$—$C_4$)-Alkyl bedeuten.

Besonders bevorzugte Verbindungen der allgemeinen Formel I sind solche, in denen

R: Chlor, Brom
n: 0, 1
A: O, S
X: $-CH_2-$
$R_1$: $-CH_3$
Z: $-COOR_3$
$R_3$: H, ($C_1$—$C_6$)-Alkyl, ggf. substituiert durch ($C_1$—$C_4$)-Alkoxi, Cl, Br, oder ein Alkali- oder -Erdalkalikation bedeutet.

Die Verbindungen der allgemeinen Formel I lassen sich aus an sich bekannten, bzw. nach bekannten Verfahren hergestellten Ausgangsmaterialien herstellen. Diese Verfahren sind dadurch gekennzeichnet, daß man

a) Verbindungen der Formel II

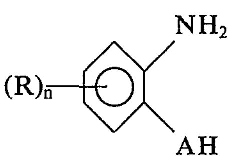

(II)

mit Verbindungen der Formel III,

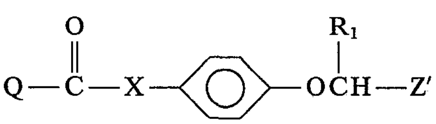

(III)

worin Q eine Hydroxygruppe, eine Alkoxygruppe oder ein Halogenatom sein kann, und Z' ein niederes Alkoxycarbonyl bedeutet, umsetzt;

b) Verbindungen der Formel IV

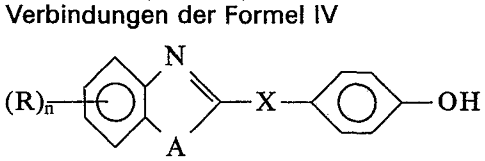

(IV)

mit Verbindungen der Formel V

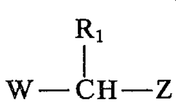

(V)

worin W Halogen oder den Rest einer aliphatischen oder aromatischen Sulfosäure bedeutet umsetzt;

c) Verbindungen der Formel I, in der Z die $-COOR_3$-Gruppe darstellt, hydriert und die erhaltenen Alkohole ($Z = CH_2OH$) gewünschtenfalls durch Umsetzung mit Carbonsäuren, Carbonsäurehalogeniden oder Anhydriden in die entsprechende Carbonsäureester

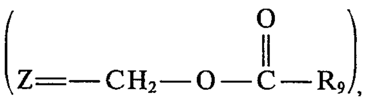

oder
durch Umsetzung mit Sulfosäurehalogeniden in Sulfosäureester ($Z = -CH_2-O-SO_2-R_{10}$) überführt;

d) Verbindungen der Formel I, in der Z die $-COOR_3$-Gruppe darstellt (worin $R_3$ nicht Wasserstoff bedeutet) in bekannter Weise in Salze (Z = COOKat) bzw. freie Säuren (Z = COOH) überführt;

e) Verbindungen der Formel I, in der Z die $-COOR_3$-Gruppe darstellt, mit Ammoniak, Aminen oder Hydrazinen zu Säureamiden ($Z = CONH\ R_5$) bzw. Säurehydraziden

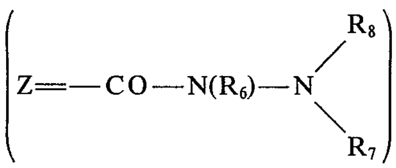

umsetzt;

f) Verbindungen der Formel I, in der Z die $-COOH$-Gruppe darstellt, durch Umsetzung zu Säurehalogeniden und anschließende Reaktion mit Aminen, Hydrazinen oder Alkoholen in Säureamide, Säurehydrazide bzw. Ester überführt;

g) Verbindungen der Formel I, in der Z eine niedermolekulare Estergruppe bedeutet, mit Alkoholen der Formel $R_3OH$ umestert;

h) Verbindungen der Formel I, in der Z die $-CO-NH_2$-Gruppe darstellt, entwässert und die erhaltenen Nitrile (Z = CN) gewünschtenfalls durch Anlagerung von $H_2S$ in die entsprechenden Thioamide ($Z = CS-NH_2$) überführt.

Zu a) Vorzugsweise wird die freie Säure (Q = OH) oder das Säurehalogenid (Q = Cl) verwendet, Z' bedeutet $-COOC_{1-6}$Alkyl.
Die Umsetzung erfolgt mit oder ohne Lösungsmittel bei Temperaturen von 100—200°C. Bevorzugte Lösungsmittel sind Xylol, Chlorbenzol, Dichlorbenzol. Das bei der Umsetzung entstehende Wasser wird abdestilliert.

Zu b) Die Umsetzung der Verbindungen IV und V erfolgt vorzugsweise in inerten aprotischen Lösungsmitteln wie aliphatischen oder aromatischen Kohlenwasserstoffen (wie z. B. Benzol, Toluol), Acetonitril, Aceton, DMSO, DMF bei Temperaturen zwischen 50°C und der Siedetemperatur des Lösungsmittels in Gegenwart von Basen wie Karbonat, Alkalihydoxid oder tertiären organischen Basen. W bedeutet Halogen oder einen Sulfonsäurerest wie Tosylat oder Mesylat.

Zu c) Die Reduktion von Säuren oder Estern zu Alkoholen wird vorzugsweise mit komplexen Metallhydriden wie $LiAlH_4$ in etherischen, wasserfreien Lösungsmitteln durchgeführt. Da die Reaktion gewöhnlich exotherm verläuft, erübrigt sich im allgemeinen eine Temperaturzufuhr von außen. Die anschließende Veresterung mit Säureanhydriden oder Säurehalogeniden erfolgt in inerten Lösungsmitteln (wie bei a)) bei Temperaturen zwischen 0°C und dem Siedepunkt des Lösungsmittels unter Zusatz einer organischen oder anorganischen Base, z. B. $Na_2CO_3$, $K_2CO_3$, Pyridin oder Triethylamin. Die Veresterung mit Carbonsäuren erfolgt entweder durch Zusatz wasserbindender Mittel wie $P_2O_5$ oder durch azeotrope extraktive Destillation der angesäuerten Komponenten.

Zu e) Zur Umsetzung von Estern der Formel I mit Aminen, Ammoniak oder Hydrazinen verwendet man vorzugsweise die gleichen Lösungsmittel wie bei b) und arbeitet bei Temperaturen zwischen 40°C und Rückflußtemperatur.

Zu f) Die Überführung von Säuren der Formel I in Säurehalogenide erfolgt in an sich bekannter Weise. Gleiches gilt für die anschließende Umsetzung mit Ammoniak, Aminen oder Hydrazinen. Zur Bindung des freiwerdenden Halogenwasserstoffs ist ein mindestens einmolarer Überschuß der eingesetzten Base erforderlich.

Zu g) Die Umsetzung geschieht durch saure oder basische Katalyse. Man setzt den Alkohol, der in den Ester eingeführt werden soll, zweckmäßigerweise im Überschuß zu und destilliert den freiwerdenden, niedriger siedenden Alkohol laufend in dem Maße ab, in dem er bei der Umesterung gebildet wird.

Zu h) Bei der Entwässerung von Amiden zu Nitrilen arbeitet man vorzugsweise in aromatischen Kohlenwasserstoffen bei Temperaturen von 50°C bis zur Siedetemperatur. Die anschließende Anlagerung von $H_2S$ erfolgt zweckmäßigerweise im Autoklaven in Gegenwart katalytischer Mengen einer Base (vorzugsweise Ethanolamin) bei Temperaturen zwischen 50 und 150°C.

Die für die Herstellung der erfindungsgemäßen Verbindungen der allgemeinen Formel I benötigten heterocyclischen Phenole der Formel IV lassen sich vorzugsweise durch Kondensation von Aminophenolen, Aminothiophenolen und Diaminobenzolen mit 4-Hydroxyphenylessigsäure bzw. 3-(4'-Hydroxyphenyl)-propionsäure herstellen. Die Kondensation erfolgt unter Abdestillieren von Wasser bei Reaktionstemperaturen von 120—200°C.

Die erfindungsgemäßen Verbindungen der allgemeinen Formel I sind im Vor- und Nachauflaufverfahren gegen ein breites Spektrum von monokotylen Schadpflanzen sehr gut wirksam, gleichzeitig werden sie jedoch von zweikeimblättrigen Kulturpflanzen sowie einigen Getreidearten vorzüglich toleriert.

Die Verbindungen sind daher u. a. zur selektiven Bekämpfung von Schadgräsern in Kulturpflanzen geeignet. Gegenstand der vorliegenden Erfindung sind daher auch herbizide Mittel, die dadurch gekennzeichnet sind, daß sie eine herbizid wirksame Menge einer Verbindung der allgemeinen Formel I neben üblichen Zusatz- und Formulierungshilfsmitteln enthalten.

Die erfindungsgemäßen Mittel enthalten die Wirkstoffe der Formel I im allgemeinen zu 2—95 Gew.-%. Sie können als benetzbare Pulver, emulgierbare Konzentrate, versprühbare Lösungen, Stäubemittel oder Granulate in den üblichen Zubereitungen angewendet werden.

Benetzbare Pulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Oleyl-, Stearylamine, Alkyl- oder Alkylphenyl-sulfonate und Dispergiermittel, z. B. ligninsulfonsaures Natrium, 2,2'-dinaphthylmethan-6,6'-disulfonsaures Natrium, oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten und Zusatz eines nichtionischen Netzmittels, beispielsweise eines polyoxethylierten Alkylphenols oder eines polyoxethylierten Oleyl- oder Stearylamins, erhalten.

Stäubemittel erhält man durch Vermahlen des Wirkstoffes mit fein verteilten, festen Stoffen, z. B. Talkum, natürlichen Tonen, wie Kaolin, Bentonit, Pyrophyllit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium, oder auch Mineralölen auf die Oberfläche von Trägerstoffen, wie Sand, Kaolinite, oder von granuliertem Inertmaterial. Auch können geeignete Wirkstoffe in der für die Herstellung von Düngemittelgranalien üblichen Weise — gewünschtenfalls in Mischung mit Düngemitteln — hergestellt werden.

Bei den herbiziden Mitteln können die Konzentrationen der Wirkstoffe in den handelsüblichen Formulierungen verschieden sein. In benetzbaren Pulvern variiert die Wirkstoffkonzentration z. B. zwischen etwa 10% und 95%, der Rest besteht aus den oben angegebenen Formulierungszusätzen. Bei emulgierbaren Konzentraten ist die Wirkstoffkonzentration etwa 10% bis 80%. Staubförmige Formulierungen enthalten meistens 5—20% an Wirkstoff. Bei Granulaten hängt der Wirkstoffgehalt z. T. davon ab, ob die wirksame Verbindung flüssig oder fest vorliegt und welche Granulierhilfsmittel, Füllstoffe usw. verwendet werden.

Zur Anwendung werden die handelsüblichen Konzentrate gegebenenfalls in üblicher Weise verdünnt, z. B. bei benetzbaren Pulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit u. a. variiert die erforderliche Aufwandmenge. Sie kann innerhalb weiter Grenzen schwanken, z. B. zwischen 0,05 und 10,0 kg/ha oder mehr Aktivsubstanz, vorzugsweise liegt sie jedoch zwischen 0,1 und 5 kg/ha.

Die erfindungsgemäße Wirkstoffe können mit anderen Herbiziden, Insektiziden und Fungiziden kombiniert werden.

## Herstellungsbeispiele

### Beispiel 1

2-[4'-(2"-Benzthiazolylmethyl)-phenoxi]-propionsäureethylester

24,1 g (0,1 Mol) 4-(2'-Benzthiazolylmethyl)-phenol werden mit 16,6 g (0,12 Mol) Kaliumkarbonat und 19,9 g (0,11 Mol) 2-Brompropionsäureethylester in 100 ml Acetonitril 8 Stunden unter Rückfluß erhitzt. Anorganische Salze werden abfiltriert, Acetonitril unter reduziertem Druck abdestilliert und der

Rückstand im Hochvakuum (Siedepunkt 205–208°C, 0,07 mbar) destilliert. Man erhält 30 g ≙ 88% d. Th. an 2-[4'-(2"-Benzthiazolylmethyl)-phenoxi]-propionsäureethylester.

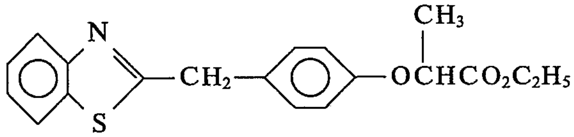

Beispiel 2

2-[4'-(2"-Benzthiazolylmethyl)-phenoxi]-propionsäure

34,1 g (0,1 Mol) 2-[4'-(2"-Benzthiazolylmethyl)-phenoxi]-propionsäureethylester werden mit 4,4 g (0,11 Mol) Natriumhydroxid 200 ml Wasser und 50 ml Methanol 16 Stunden bei Raumtemperatur gerührt. Anschließend wird mit 15 ml konz. Salzsäure angesäuert und der entstandene Niederschlag abfiltriert und getrocknet. Man erhält 30 g = 96% d. Th. 2-[4'-(2"-Benzthiazolylmethylen)-phenoxi]-propionsäure vom Schmelzpunkt 154–156°C.

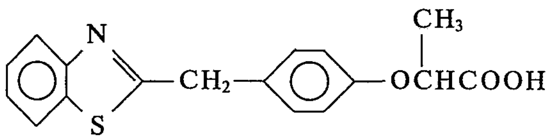

In analoger Weise erhält man:

| Beispiel Nr. | Verbindung | gemäß Beispiel | Kp./Fp./$n_D$ |
|---|---|---|---|
| 3 | 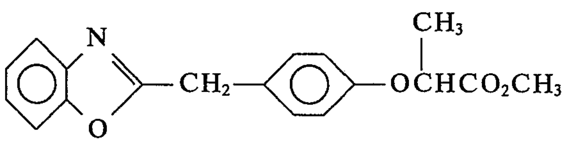 | 1 | Fp. 63°C |
| 4 | 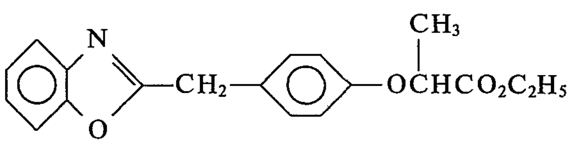 | 1 | Kp. 174–176°C, 0,013 mbar |
| 5 | 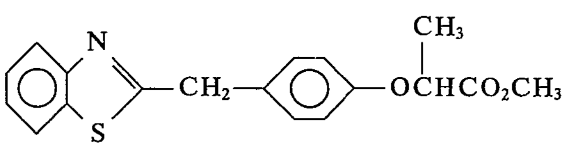 | 1 | Fp. 70°C |
| 6 | 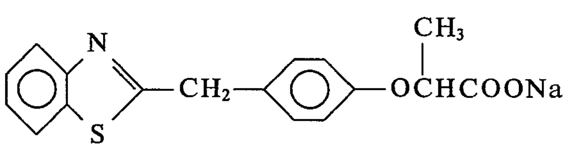 | 2 | glasig, zäh |
| 7 | 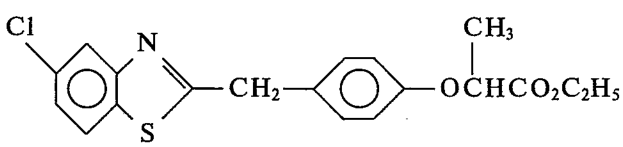 | 1 | |

Fortsetzung

| Bei-spiel Nr. | Verbindung | gemäß Bei-spiel | Kp./Fp./$n_D$ |
|---|---|---|---|
| 8 | 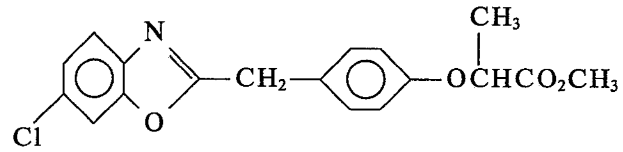 | 1 | Kp. 192°C, 0,007 mbar |
| 9 | 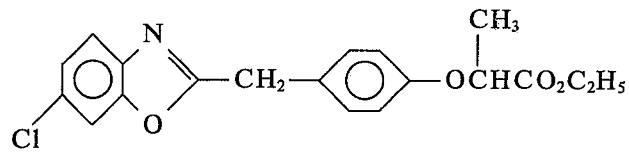 | 1 | |
| 10 | 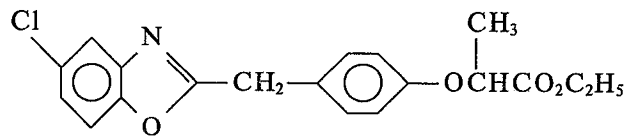 | 1 | Kp. 181°C, 0,005 mbar |
| 11 | 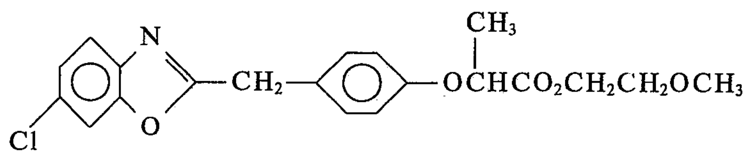 | 1 | |
| 12 | 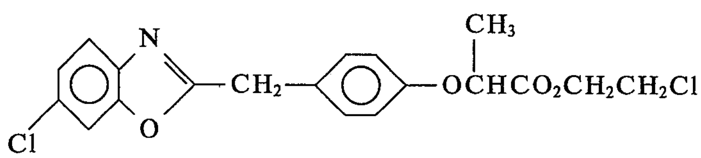 | 1 | |
| 13 | 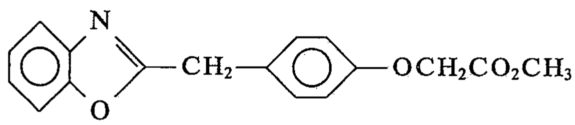 | 1 | Fp. 91–93°C |
| 14 | 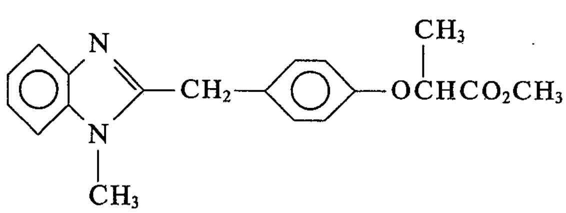 | 1 | Kp.: 226°C, 0,08 mbar |
| 15 | 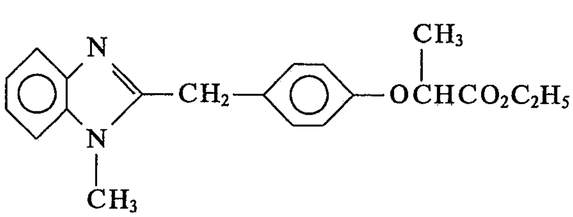 | 1 | Kp.: 206°C, 0,07 mbar |

Fortsetzung

| Bei-spiel Nr. | Verbindung | gemäß Beispiel | Kp./Fp./$n_D$ |
|---|---|---|---|
| 16 | 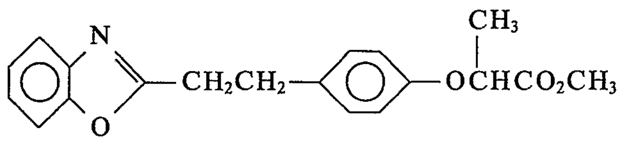 | 1 | Fp.: 65–66°C |
| 17 | 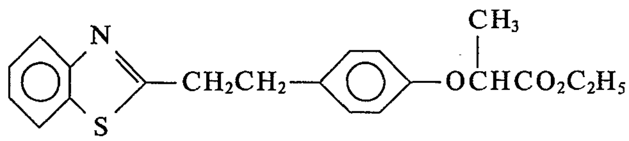 | 1 | Kp.: 198°C, 0,01 mbar |
| 18 | 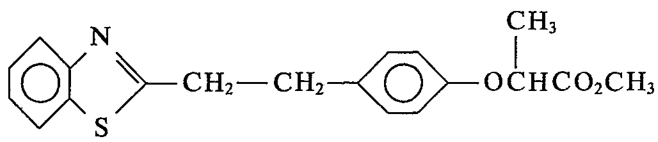 | 1 | Kp.: 190°C, 0,007 mbar |
| 19 | 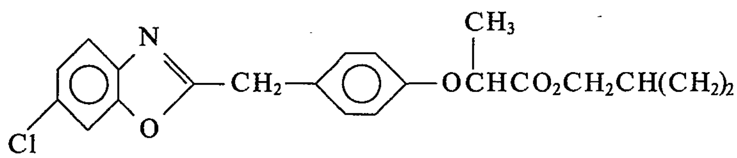 | 1 | |

## Formulierungsbeispiele

### Beispiel A

Ein emulgierbares Konzentrat wird erhalten aus
- 15 Gew.-Teilen Wirkstoff
- 75 Gew.-Teilen Cyclohexanon als Lösungsmittel und
- 10 Gew.-Teilen oxethyliertes Nonylphenol (10 AeO) als Emulgator.

### Beispiel B

Ein in Wasser leicht dispergierbares benetzbares Pulver wird erhalten, indem man
- 25 Gew.-Teile Wirkstoff
- 64 Gew.-Teile kaolinhaltiges Quarz als Inertstoff
- 10 Gew.-Teile ligninsulfonsaures Kalium und
- 1 Gew.-Teil oleylmethyltaurinsaures Natrium als Netz- und Dispergiermittel

mischt und in einer Stiftmühle mahlt.

### Beispiel C

Ein Stäubemittel wird erhalten, indem man
- 10 Gew.-Teile Wirkstoff und
- 90 Gew.-Teile Talkum als Inertstoff

mischt und in einer Schlagmühle zerkleinert.

### Beispiel D

Ein Granulat besteht z. B. aus etwa
- 2–15 Gew.-Teilen Wirkstoff
- 98–85 Gew.-Teilen inerten Granulatmaterialien, wie z. B. Attapulgit, Bimsstein und Quarzsand.

## Anwendungsbeispiele

### Beispiel 1

Verschiedene wirtschaftlich wichtige Ungräser und Kulturpflanzen wurden in Töpfen ausgesät und vor dem Auflaufen mit den erfindungsgemäßen Verbindungn behandelt. Die Töpfe wurden anschließend im Gewächshaus unter guten Wachstumsbedingungen gehalten. 4 Wochen später wurden die Wirksamkeit gegen die Ungräser und das Selektivitätsverhalten an den Kulturpflanzen visuell auf prozentuale Schädigung im Vergleich zu unbehandelten Pflanzen bonitiert. Hierbei ergaben sich die in Tabelle 1 dargestellten Werte, die belegen, daß die erfindungsgemäßen Verbindungen gegen viele Ungräser gut herbizid wirksam sind und gleichzeitig in hohen Dosierungen von z. B. 2,4 kg AS/ha Kulturpflanzen nicht oder nur ganz geringfügig schädigen. Sie eignen sich daher zur selektiven Unkrautbekämpfung in solchen Kulturen.

### Beispiel 2

In ähnlicher Weise wurden die erfindungsgemäßen Verbindungen im Nachauflaufverfahren auf einige wichtige Ungrasarten und verschiedene Kulturpflanzen gesprüht, die im Gewächshaus bis zur Größe von 8–15 cm angezogen worden waren. Nach weiteren 4 Wochen Standzeit im Gewächshaus wurden die Pflanzen hinsichtlich ihrer prozentualen Schädigung durch die Herbizid-Behandlungen bonitiert. Die Ergebnisse, dargestellt in Tabelle 2, belegen, daß erfindungsgemäße Verbindungen auch im Nachauflaufverfahren zur selektiven Bekämpfung von Ungräsern in zahlreichen Kulturen dikotyler und monokotyler Art, wie z. B. Baumwolle oder Reis, eingesetzt werden können.

Tabelle 1

Wirkung und Selektivität der Verbindungen im Vorauflaufverfahren

| Beispiel Nr. | kg AS/ha | AL | Wirksamkeit gegen ECG | in % SA | Selektivität in Kulturpflanzen (Schädigung in %) GH | GS | BA |
|---|---|---|---|---|---|---|---|
| 10 | 2,4 | 100 | 100 | 100 | 0 | 2 | 5 |
| | 0,6 | 100 | 100 | 95 | 0 | 0 | 0 |
| 2 | 2,4 | 98 | 99 | 95 | 4 | 5 | 4 |
| | 0,6 | 95 | 98 | 90 | 0 | 0 | 0 |
| 3 | 2,4 | 85 | 90 | 90 | 0 | 0 | 0 |
| 4 | 2,4 | 85 | 90 | 75 | 0 | 0 | 0 |
| 1 | 2,4 | 99 | 99 | 95 | 0 | 0 | 0 |
| | 0,6 | 98 | 95 | 90 | – | – | – |
| 5 | 2,4 | 100 | 100 | 95 | 0 | 0 | 0 |
| | 0,6 | 98 | 95 | 92 | – | – | – |

AL = Alopecurus
ECG = Echinochloa
SA = Setaria
GH = Baumwolle
GS = Sojabohne
BA = Zuckerrübe

Tabelle 2

Herbizide Wirksamkeit und Selektivität im Nachauflaufverfahren (Schädigung in %)

| Beispiel Nr. | kg AS/ha | AL | ECG | SA | GH | GS | BA | HV | OS |
|---|---|---|---|---|---|---|---|---|---|
| 10 | 2,4 | 100 | 99 | 100 | 0 | 2 | 1 | 7 | 4 |
| | 0,6 | 96 | 99 | 99 | 0 | 0 | 0 | 1 | 2 |
| 5 | 2,4 | 96 | 95 | 95 | 0 | 2 | 0 | – | – |
| | 0,6 | 92 | 90 | – | 0 | 0 | 0 | – | – |

HV = Gerste
OS = Reis

**Patentansprüche für die Vertragsstaaten: BE, CH, DE, FR, GB, IT, LU, NL, SE**

1. Verbindungen der Formel I

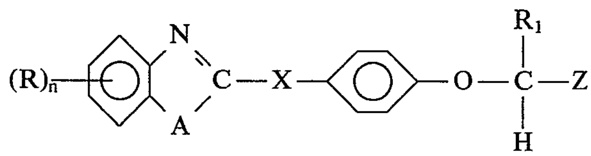

(I)

worin

R: Halogen, $CF_3$, $NO_2$, CN, $NH_2$, $(C_1-C_4)$-Alkyl, $(C_1-C_4)$-Alkoxi, $(C_1-C_4)$-Alkylthio oder Phenoxi, das gegebenenfalls ein- bis dreifach durch Halogen, $CF_3$, $(C_1-C_4)$-Alkyl, $NO_2$ oder die Gruppe $-NH-C(O)-R_2$ substituiert sein kann;
n: 0, 1, 2, 3 oder 4,
A: O, S, NH oder N-$(C_1-C_4)$-Alkyl,
X: $-CH_2-$ oder $-CH_2-CH_2-$,
$R_1$: H oder $(C_1-C_4)$-Alkyl
Z: eine Gruppe der Formel

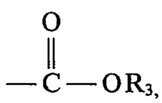, 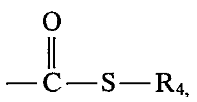, 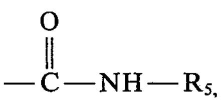, 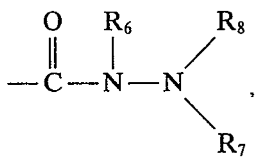, 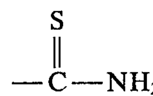, CN,

$-CH_2OH$, 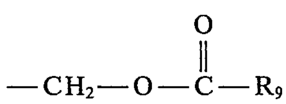 oder $-CH_2-O-SO_2-R_{10}$,

$R_3$: H, $(C_1-C_{12})$-Alkyl, das gegebenenfalls durch 1–6 Halogen, vorzugsweise F, Cl, Br und/oder durch OH, $(C_1-C_6)$-Alkoxi, $(C_1-C_4)$-Alkylthio, $(C_1-C_6)$-Alkoxi-$(C_2-C_6)$-alkoxi, Halogen-$(C_1-C_2)$-alkoxi, Methoxi-ethoxi-ethoxi, $(C_1-C_4)$-Alkylamino, Di-$(C_1-C_4)$-alkylamino, $(C_1-C_4)$-Alkoxicarbonyl. Phenyl, Oxiranyl und Phenoxi substituiert ist, wobei letzteres ebenfalls ein- bis zweifach durch Halogen oder $(C_1-C_4)$-Alkyl substituiert sein kann;
$(C_5-C_6)$-Cycloalkyl oder Halogen-$(C_5-C_6)$-alkyl;
$(C_3-C_6)$-Alkenyl, Halogen-$(C_3-C_6)$-alkenyl oder $(C_5-C_6)$-Cycloalkenyl;
$(C_3-C_4)$-Alkinyl, das gegebenenfalls ein- oder zweifach durch $(C_1-C_6)$-Alkyl, Phenyl, Halogen bzw. $(C_1-C_2)$-Alkoxi substituiert ist;
Phenyl, das gegebenenfalls ein- bis dreifach durch $(C_1-C_4)$-Alkyl, $(C_1-C_4)$-Alkoxi, Halogen, $NO_2$ oder $CF_3$ substituiert ist; Furfuryl, Tetrahydrofurfuryl oder
ein Kationäquivalent einer organischen oder anorganischen Base ist;
$R_4$: $(C_1-C_6)$-Alkyl, das gegebenenfalls durch $(C_1-C_4)$-Alkoxi, Halogen oder Phenyl substituiert ist, wobei letzteres ebenfalls ein- bis dreifach durch $(C_1-C_4)$-Alkyl und Halogen substituiert sein kann; $(C_3-C_6)$-Alkenyl oder Phenyl, das gegebenenfalls ein- bis dreifach durch $(C_1-C_4)$-Alkyl und/ oder Halogen substituiert ist,

$R_5$: H, ($C_1$—$C_6$)-Alkyl, Hydroxy-($C_1$—$C_6$)-Alkyl oder ($C_5$—$C_6$)-Cycloalkyl,
$R_6$: H oder $CH_3$,
$R_7$: H, $CH_3$ oder $C_2H_5$,
$R_8$: H, $CH_3$, $C_2H_5$ oder Phenyl,
$R_9$: ($C_1$—$C_6$)-Alkyl, das gegebenenfalls ein- bis dreifach durch Halogen substituiert ist, $C_3$-, $C_5$- oder $C_6$-Cycloalkyl, ($C_3$—$C_6$)-Alkenyl, Phenyl, ($C_1$—$C_4$)-Alkylphenyl, ($C_1$—$C_4$)-Alkoxiphenyl, Halogenphenyl, Trifluormethylphenyl oder Nitrophenyl sowie
$R_{10}$: ($C_1$—$C_4$)-Alkyl oder Phenyl, das gegebenenfalls ein- bis dreifach durch Halogen, $CF_3$, $NO_2$ oder ($C_1$—$C_4$)-Alkyl substituiert ist, bedeuten.

2. Verfahren zur Herstellung von Verbindungen der Formel I, dadurch gekennzeichnet, daß man

a) Verbindungen der Formel II

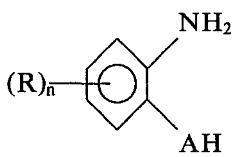

(II)

mit Verbindungen der Formel III,

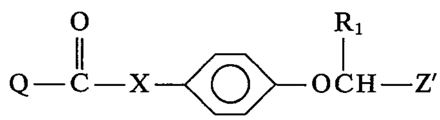

(III)

worin Q eine Hydroxygruppe, eine Alkoxygruppe oder ein Halogenatom sein kann, und Z' ein niederes Alkoxycarbonyl bedeutet, umsetzt;

b) Verbindungen der Formel IV

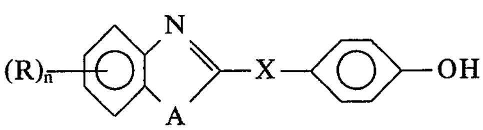

(IV)

mit Verbindungen der Formel V

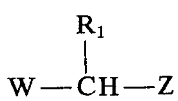

(V)

worin W Halogen oder den Rest einer aliphatischen oder aromatischen Sulfosäure bedeutet, umsetzt;

c) Verbindungen der Formel I, in der Z die $-COOR_3$-Gruppe darstellt, hydriert und die erhaltenen Alkohole (Z = $CH_2OH$) gewünschtenfalls durch Umsetzung mit Carbonsäuren, Carbonsäurehalogeniden oder Anhydriden in die entsprechenden Carbonsäureester

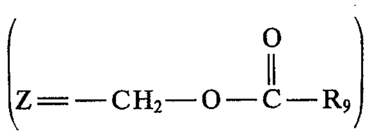

durch Umsetzung mit Sulfonsäurehalogeniden in Sulfosäureester (Z = $-CH_2-O-SO_2-R_{10}$) überführt;

d) Verbindungen der Formel I, in der Z die $-COOR_3$-Gruppe darstellt (worin $R_3$ nicht Wasserstoff bedeutet) in bekannter Weise in Salze (Z = COOKat) bzw. freie Säuren (Z = COOH) überführt;

e) Verbindungen der Formel I, in der Z die $-COOR_3$-Gruppe darstellt, mit Ammoniak, Aminen oder Hydrazinen zu Säureamiden (Z = CONH—$R_5$) bzw. Säurehydraziden

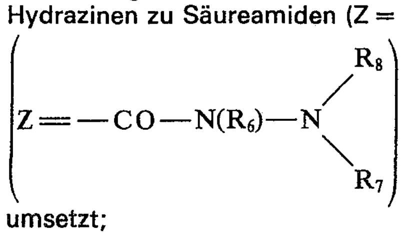

umsetzt;

f) Verbindungen der Formel I, in der Z die —COOH-Gruppe darstellt, durch Umsetzung zu Säurehalogeniden und anschließende Reaktion mit Aminen, Hydrazinen oder Alkoholen in Säureamide, Säurehydrazide bzw. Ester überführt;

g) Verbindungen der Formel I, in der Z eine niedermolekulare Estergruppe bedeutet, mit Alkoholen der Formel $R_3OH$ umestert;

h) Verbindungen der Formel I, in der Z die $—CO—NH_2$-Gruppe darstellt, entwässert und die erhaltenen Nitrile (Z = CN) gewünschtenfalls durch Anlagerung von $H_2S$ in die entsprechenden Thioamide (Z = $CS—NH_2$) überführt.

3. Herbizide Mittel, gekennzeichnet durch einen Gehalt an einer Verbindung der Formel I.
4. Verwendung von Verbindungen der Formel I zur Bekämpfung von monokotylen Schadpflanzen.
5. Verbindung der Formel

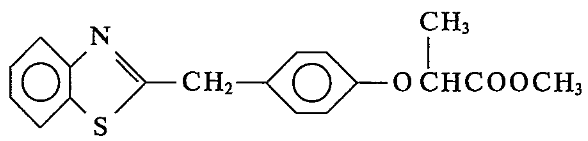

6. Verbindung der Formel

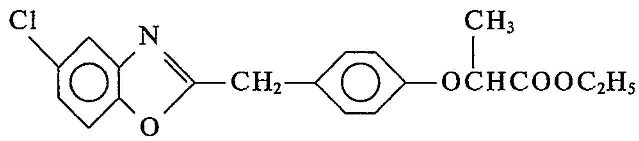

## Patentansprüche für den Vertragsstaat: AT

1. Herbizide Mittel, gekennzeichnet durch einen Gehalt an einer Verbindung der Formel I

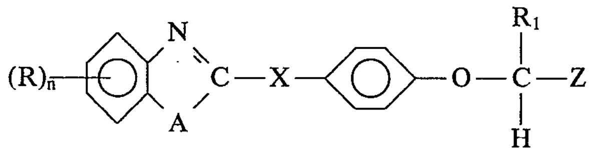

(I)

worin

R: Halogen, $CF_3$, $NO_2$, CN, $NH_2$, $(C_1—C_4)$-Alkyl, $(C_1—C_4)$-Alkoxi, $(C_1—C_4)$-Alkylthio oder Phenoxi, das gegebenenfalls ein- bis dreifach durch Halogen, $CF_3$, $(C_1—C_4)$-Alkyl, $NO_2$ oder die Gruppe $—NH—C(O)—R_2$ substituiert sein kann;
n: 0, 1, 2, 3 oder 4
A: O, S, NH oder N-$(C_1—C_4)$-Alkyl,
X: $—CH_2—$ oder $—CH_2—CH_2—$,
$R_1$: H oder $(C_1—C_4)$-Alkyl
Z: eine Gruppe der Formel

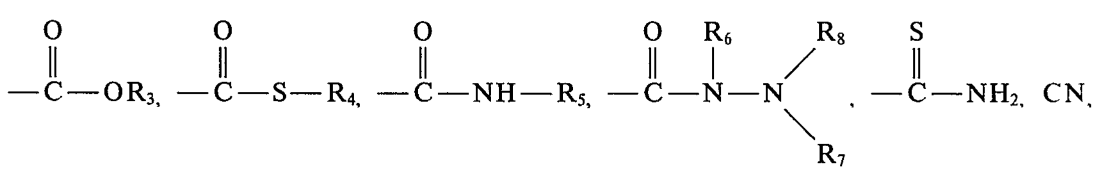

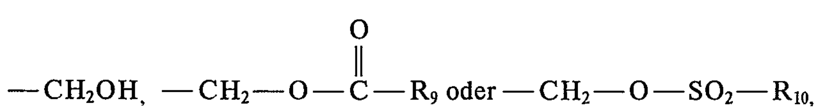

$R_3$: H, $(C_1—C_{12})$-Alkyl, das gegebenenfalls durch 1—6 Halogen, vorzugsweise F, Cl, Br und/oder durch OH, $(C_1—C_6)$-Alkoxi, $(C_1—C_4)$-Alkylthio, $(C_1—C_6)$-Alkoxi-$(C_2—C_6)$-alkoxi, Halogen-$(C_1—C_2)$-alkoxi, Methoxi-ethoxi-ethoxi, $(C_1—C_4)$-Alkylamino, Di-$(C_1—C_4)$-alkylamino, $(C_1—C_4)$-Alkoxicarbonyl, Phenyl, Oxiranyl und Phenoxi substituiert ist, wobei letzteres ebenfalls ein- bis zweifach durch

Halogen oder ($C_1$—$C_4$)-Alkyl substituiert sein kann:
($C_5$—$C_6$)-Cycloalkyl oder Halogen-($C_5$—$C_6$)-alkyl;
($C_3$—$C_6$)-Alkenyl, Halogen-($C_3$—$C_6$)-alkenyl oder ($C_5$—$C_6$)-Cycloalkenyl;
($C_3$—$C_4$)-Alkinyl, das gegebenenfalls ein- oder zweifach durch ($C_1$—$C_6$)-Alkyl, Phenyl, Halogen bzw. ($C_1$—$C_2$)-Alkoxi substituiert ist;
Phenyl, das gegebenenfalls ein- bis dreifach durch ($C_1$—$C_4$)-Alkyl, ($C_1$—$C_4$)-Alkoxi, Halogen, $NO_2$ oder $CF_3$ substituiert ist; Furfuryl, Tetrahydrofurfuryl oder ein Kationäquivalent einer organischen oder anorganischen Base ist;

$R_4$: ($C_1$—$C_6$)-Alkyl, das gegebenenfalls durch ($C_1$—$C_4$)-Alkoxi, Halogen oder Phenyl substituiert ist, wobei letzteres ebenfalls ein- bis dreifach durch ($C_1$—$C_4$)-Alkyl und Halogen substituiert sein kann; ($C_3$—$C_6$)-Alkenyl oder Phenyl, das gegebenenfalls ein- bis dreifach durch ($C_1$—$C_4$)-Alkyl und/oder Halogen substituiert ist;
$R_5$: H, ($C_1$—$C_6$)-Alkyl, Hydroxy-($C_1$—$C_6$)-Alkyl oder ($C_5$—$C_6$)-Cycloalkyl;
$R_6$: H oder $CH_3$;
$R_7$: H, $CH_3$ oder $C_2H_5$;
$R_8$: H, $CH_3$, $C_2H_5$ oder Phenyl;
$R_9$: ($C_1$—$C_6$)-Alkyl, das gegebenenfalls ein- bis dreifach durch Halogen substituiert ist, $C_3$-, $C_5$- oder $C_6$-Cycloalkyl, ($C_3$—$C_6$)-Alkenyl, Phenyl, ($C_1$—$C_4$)-Alkylphenyl, ($C_1$—$C_4$)-Alkoxiphenyl, Halogenphenyl, Trifluormethylphenyl oder Nitrophenyl sowie
$R_{10}$: ($C_1$—$C_4$)-Alkyl oder Phenyl, das gegebenenfalls ein- bis dreifach durch Halogen, $CF_3$, $NO_2$ oder ($C_1$—$C_4$)-Alkyl substituiert ist, bedeuten.

2. Verwendung von Verbindungen der Formel I zur Bekämpfung von monokotylen Schadpflanzen.

**Claims for the Contracting states: BE, CH, DE, FR, GB, IT, LU, NL, SE**

1. Compounds of the formula I

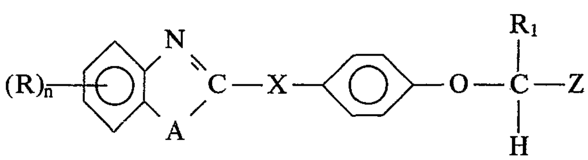

(I)

in which
R is halogen, $CF_3$, $NO_2$, CN, $NH_2$, ($C_1$—$C_4$)alkyl, ($C_1$—$C_4$)alkoxy, ($C_1$—$C_4$)alkylthio, or phenoxy optionally mono-, di- or tri-substituted by halogen, $CF_3$, ($C_1$—$C_4$)alkyl, $NO_2$ or —NH—CO—$R_2$
n is zero, or an integer from 1 to 4,
A is O, S, NH, or N—($C_1$—$C_4$)alkyl,
X is $-CH_2-$ or $-CH_2-CH_2$,
$R_1$ is H or ($C_1$—$C_4$)alkyl,
Z is a group of the formula

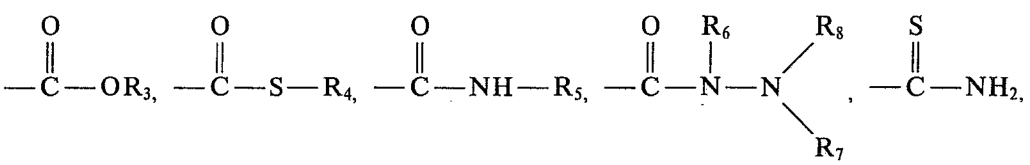

CN,

$-CH_2OH$, 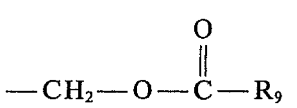 or $-CH_2-O-SO_2-R_{10}$,

$R_3$ is H, ($C_1$—$C_{12}$)alkyl optionally substituted by 1 to 6 halogen atoms, preferably F, Cl, Br, and/or by OH, ($C_1$—$C_6$)alkoxy, ($C_1$—$C_4$)alkylthio, ($C_1$—$C_6$)alkoxy-($C_2$—$C_6$)alkoxy, halo($C_1$—$C_2$)alkoxy, methoxyethoxy-ethoxy, ($C_1$—$C_4$)alkylamino, di($C_1$—$C_4$)alkylamino, ($C_1$—$C_4$)alkoxycarbonyl, phenyl, oxiranyl and phenoxy which in turn is optionally mono- or disubstituted by halogen or ($C_1$—$C_4$)alkyl, ($C_5$—$C_6$)cycloalkyl or halo($C_5$—$C_6$)cyclo-alkyl;
($C_3$—$C_6$)alkenyl, halo($C_3$—$C_6$)alkenyl, or ($C_5$—$C_6$)cycloalkenyl;
($C_3$—$C_4$)alkinyl optionally being mono- or disubstituted by ($C_1$—$C_6$)alkyl, phenyl, halogen, or ($C_1$—$C_2$)alkoxy;
phenyl optionally mono-, di-, or trisubstituted by ($C_1$—$C_4$)alkyl, ($C_1$—$C_4$)alkoxy, halogen, $NO_2$, or $CF_3$; furfuryl, tetrahydrofurfuryl, or

a cation equivalent of an organic or inorganic base;

$R_4$ is $(C_1-C_6)$alkyl optionally substituted by $(C_1-C_4)$alkoxy, halogen or phenyl which in turn may be mono-, di- or trisubstituted by $(C_1-C_4)$alkyl and halogen; $(C_3-C_6)$alkenyl or phenyl which is optionally mono-, di- or trisubstituted by $(C_1-C_4)$alkyl and/or halogen;

$R_5$ is H, $(C_1-C_6)$alkyl, hydroxy$(C_1-C_6)$alkyl, or $(C_5-C_6)$cycloalkyl;

$R_6$ is H or $CH_3$;

$R_7$ is H, $CH_3$ or $C_2H_5$;

$R_8$ is H, $CH_3$, $C_2H_5$, or phenyl;

$R_9$ is $(C_1-C_6)$alkyl, $(C_1-C_6)$haloalkyl, $C_3$-, $C_5$-, or $C_6$-cycloalkyl, $(C_3-C_6)$alkenyl, phenyl, $(C_1-C_4)$-alkylphenyl, $(C_1-C_4)$alkoxyphenyl, halophenyl, trifluormethylphenyl, or nitrophenyl; and

$R_{10}$ is $(C_1-C_4)$alkyl or phenyl which is optinally mono-, di-, or trisubstituted by halogen, $CF_3$, $NO_2$, or $(C_1-C_4)$alkyl.

2. Process for the manufacture of compounds of formula I which comprises

a) reacting a compound of the formula II

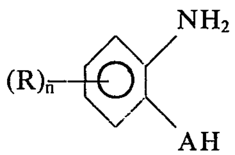

(II)

with a compound of the formula III

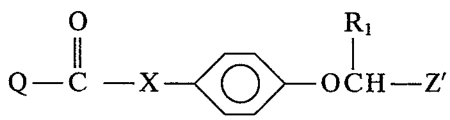

(III)

in which Q is hydroxy, alkoxy or halogen and Z' is lower alkoxycarbonyl;

b) reacting a compound of the formula IV

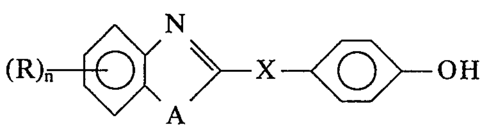

(IV)

with a compound of the formula V

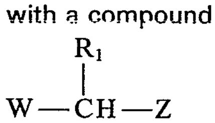

(V)

in which W is halogen or the radical of an aliphatic or aromatic sulfonic acid;

c) hyrogenating a compound of the formula I in which Z is $-COOR_3$ and optionally converting the alcohol obtained ($Z = CH_2OH$) into the corresponding carboxylic acid ester

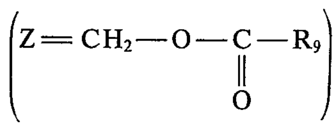

by reaction with a carboxylic anhydride, or into the sulfonic acid ester ($Z = -CH_2-O-SO_2-R_{10}$) by reaction with a sulfonic acid halide;

d) converting a compound of the formula I in which Z is $-COOR_3$ in which $R_3$ is not hydrogen in known manner into a salt (Z = COOcat) or the free acid (Z = COOH);

e) reacting a compound of the formula I in which Z is $-COOR^3$ with ammonia, an amine or hydrazine to give the acid amide ($Z = CONHR_5$) or acid hydrazide

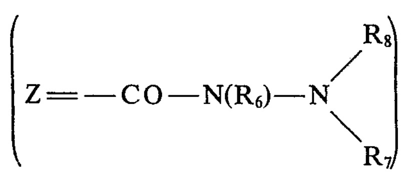

f) reacting a compound of the formula I in which Z is $-COOH$ to give the acid halide and converting the latter into the acid amide, acid hydrazide or ester by reaction with an amine, a hydrazine or an alcohol;

g) transesterifying a compound of the formula I in which Z is a low molecular weight ester group with an alcohol of the formula $R_3OH$;

h) dehydrating a compound of the formula I in which Z is $-CO-NH_2$ and converting the nitrile obtained ($Z = CN$), if desired, into the corresponding thioamide ($Z = CS-NH_2$) by addition of $H_2S$.

3. Herbicidal composition containing as active substance a compound as claimed in claim 1.
4. The use of compounds of Formula I for the combatting of monocotyledonous weeds.
5. A compound which is

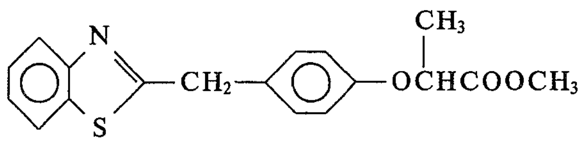

6. A compound which is

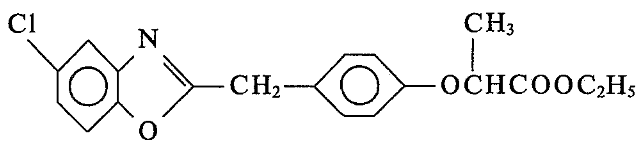

**Claims for the Contracting state: AT**

1. Herbicidal agents containing a compound of the formula I

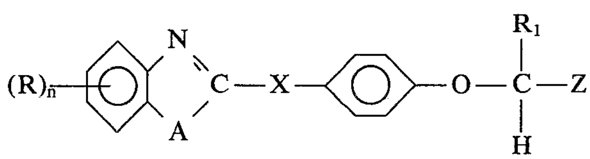

(I)

in which

R is halogen, $CF_3$, $NO_2$, CN, $NH_2$, $(C_1-C_4)$alkyl, $(C_1-C_4)$alkoxy, $(C_1-C_4)$alkylthio, or phenoxy optionally mono-, di- or tri-substituted by halogen, $CF_3$, $(C_1-C_4)$alkyl, $NO_2$ or $-NH-CO-R_2$

n is zero, or an integer from 1 to 4,

A is O, S, NH, or N-$(C_1-C_4)$alkyl,

X is $-CH_2-$ or $-CH_2-CH_2$,

$R_1$ is H or $(C_1-C_4)$alkyl,

Z is a group of the formula

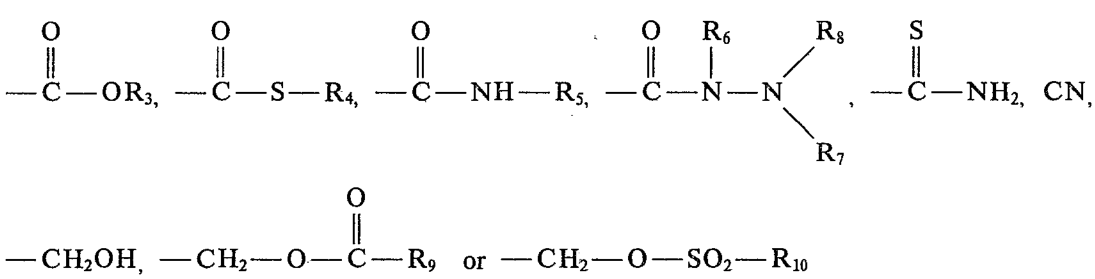

$R_3$ is H, $(C_1-C_{12})$alkyl optionally substituted by 1 to 6 halogen atoms, preferably F, Cl, Br, and/or by OH, $(C_1-C_6)$alkoxy, $(C_1-C_4)$alkylthio, $(C_1-C_6)$alkoxy-$(C_2-C_6)$alkoxy, halo$(C_1-C_2)$alkoxy, methoxyethoxy-ethoxy, $(C_1-C_4)$alkylamino, di$(C_1-C_4)$alkylamino, $(C_1-C_4)$alkoxycarbonyl, phenyl, oxiranyl and phenoxy which in turn is optionally mono- or disubstituted by halogen or $(C_1-C_4)$alkyl, $(C_5-C_6)$cycloalkyl or halo$(C_5-C_6)$cyclo-alkyl;
$(C_3-C_6)$alkenyl, halo$(C_3-C_6)$alkenyl, or $(C_5-C_6)$cycloalkenyl;
$(C_3-C_4)$alkinyl optionally being mono- or disubstituted by $(C_1-C_6)$alkyl, phenyl, halogen, or $(C_1-C_2)$alkoxy; phenyl optionally mono-, di-, or trisubstituted by $(C_1-C_4)$alkyl, $(C_1-C_4)$alkoxy,

halogen, $NO_2$, or $CF_3$; furfuryl, tetrahydrofurfuryl, or *a cation equivalent of an organic or inorganic base;*

$R_4$ is $(C_1-C_6)$alkyl optionally substituted by $(C_1-C_4)$alkoxy, halogen or phenyl which in turn may be mono-, di- or trisubstituted by $(C_1-C_4)$alkyl and halogen; $(C_3-C_6)$alkenyl or phenyl which is optionally mono-, di- or trisubstituted by $(C_1-C_4)$alkyl and/or halogen;

$R_5$ is H, $(C_1-C_6)$alkyl, hydroxy$(C_1-C_6)$alkyl, or $(C_5-C_6)$cycloalkyl;

$R_6$ is H or $CH_3$;

$R_7$ is H, $CH_3$ or $C_2H_5$;

$R_8$ is H, $CH_3$, $C_2H_5$, or phenyl;

$R_9$ is $(C_1-C_6)$alkyl, $(C_1-C_6)$haloalkyl, $C_3$-, $C_5$-, or $C_6$-cycloalkyl, $(C_3-C_6)$alkenyl, phenyl, $(C_1-C_4)$-alkylphenyl, $(C_1-C_4)$alkoxyphenyl, halophenyl, trifluoromethylphenyl, or nitrophenyl; and

$R_{10}$ is $(C_1-C_4)$alkyl or phenyl which is optinally mono-, di-, or trisubstituted by halogen, $CF_3$, $NO_2$, or $(C_1-C_4)$alkyl.

2. The use of compounds of Formula I for the combatting of monocotyledonous weeds.

**Revendications pour les Etats contractants: BE, CH, DE, FR, GB, IT, LU, NL, SE**

1. Composés répondant à la formule I:

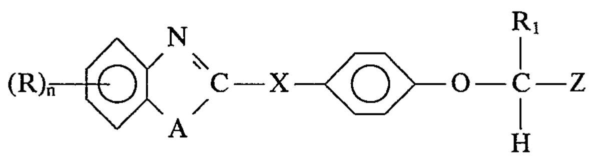

(I)

dans laquelle

R représente un halogène, un radical $CF_3$, $NO_2$, CN, $NH_2$, alkyle en $C_1-C_4$, alcoxy en $C_1-C_4$, alkylthio en $C_1-C_4$, ou un radical phenoxy éventuellement porteur d'un à trois substituants pris dans l'ensemble constitué par les halogènes, $CF_3$, les alkyles en $C_1-C_4$, $NO_2$ et les radicaux $-NH-C(O)-R_2$,

n représente un nombre égal à 0, 1, 2, 3 ou 4

A représente O, S, NH ou un radical N-Alkyl en $C_1-C_4$,

X représente $-CH_2-$ ou $-CH_2-CH_2-$,

$R_1$ représente H ou un alkyle en $C_1-C_4$,

Z représente un radical répondant à l'une des formules suivantes:

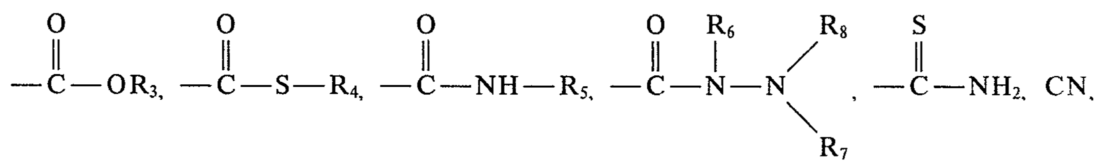

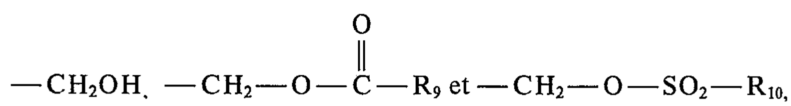

$R_3$ représente:

- un atome d'hydrogéne,
- un alkyle en $C_1-C_{12}$ qui porte éventuellement de l à 6 atomes d'halogènes, de préférence F, Cl ou Br, et/ou un radical OH, alcoxy en $C_1-C_6$, alkylthio en $C_1-C_4$, $(C_1-C_6)$-alcoxy-$(C_2-C_6)$-alcoxy, halogéno-alcoxy en $C_1$ ou $C_2$, méthoxy-ethoxy-ethoxy, alkylamino en $C_1-C_4$, di-$(C_1-C_4)$-alkylamino, $(C_1-C_4)$-alcoxycarbonyle, phényle, oxirannyle ou phénoxy, ce dernier pouvant lui-même porter un ou deux substituants pris dans l'ensemble constitué par les halogènes et les alkyles en $C_1-C_4$,
- un cycloalkyle en $C_5$ ou $C_6$ ou un halogenoalkyle en $C_5$ ou $C_6$,
- un alcényle en $C_3-C_6$, un halogénoalcényle en $C_3-C_6$, ou un cycloalcényle en $C_5$ ou $C_6$,
- un alcynyle en $C_3$ ou $C_4$, lequel porte éventuellement un ou deux substituants pris dans l'ensemble constitué par les alkyles en $C_1-C_6$, le phényle, les halogènes et les alcoxy en $C_1$ et $C_2$,
- un phényle éventuellement porteur d'un à trois substituants pris dans l'ensemble constitué par les alkyles en $C_1-C_4$, les alcoxy en $C_1-C_4$, les halogènes, $NO_2$ et $CF_3$,
- un furfuryle ou un tétrahydrofurfuryle, ou
- un équivalent cationique d'une base minérale ou organique,

$R_4$ représente:
- un alkyle en $C_1$—$C_6$, éventuellement porteur d'un alcoxy en $C_1$—$C_4$, d'un halogène ou d'un phényle, ce dernier pouvant, lui-même porter de un à trois substituants pris dans l'ensemble constitué par les alkyles en $C_1$—$C_4$ et les halogènes,
- un alcényle en $C_3$—$C_6$, ou
- un phényle éventuellement porteur d'un à trois substituants pris dans l'ensemble constitué par les alkyles en $C_1$—$C_4$ et les halogènes,

$R_5$ représente:
H, un alkylène en $C_1$—$C_6$, un hydroxyalkyle en ($C_1$—$C_6$) ou un cycloalkyle en $C_5$ ou $C_6$,

$R_6$ représente:
H ou $CH_3$,

$R_7$ représente:
H, $CH_3$ ou $C_2H_5$,

$R_8$ représente:
H, $CH_3$, $C_2H_5$ ou un phényle,

$R_9$ représente:
un alkyle en $C_1$—$C_6$, éventuellement porteur d'un à trois atomes d'halogènes, un cycloalkyle en $C_3$, $C_5$ ou $C_6$, un alcényle en $C_3$—$C_6$, un phényle, un alkylphényle à alkyle en $C_1$—$C_4$, un alcoxyphényle à alcoxy en ($C_1$—$C_4$), un halogénophényle, un trifluorméthyl-phényle ou un nitrophényle,

$R_{10}$ représente:
un alkyle en $C_1$—$C_4$ ou un phényle, ce dernier pouvant également porter de un à trois substituants pris dans l'ensemble constitué par les halogènes, $CF_3$, $NO_2$ et les alkyles en $C_1$—$C_4$.

2. Procédé de préparation de composés de formule I, procédé caractérisé en ce que:

a) on fait réagir des composés de formule II:

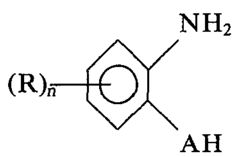

(II)

avec des composés répondant à la formule III:

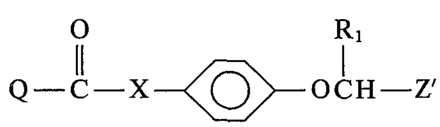

(III)

dans laquelle Q peut représenter un radical hydroxy, un radical alcoxy ou un atome d'halogène, et Z' représente un radical alcoxycarbonyle inférieur,

b) on fait réagir des composés de formule IV:

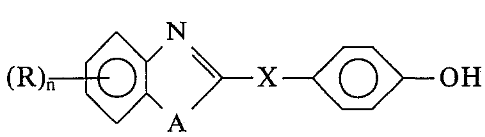

(IV)

avec des composés répondant à la formule V:

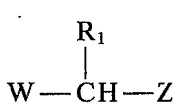

(V)

dans laquelle W représente un halogène ou le radical d'un acide sulfonique aliphatique ou aromatique,

c) on hydrogène des composés de formule I dans lesquels Z représente un radical $-COOR_3$ et, si on le désire, on transforme les alcools obtenus (Z étant alors un radical $CH_2OH$), par réaction avec des acides carboxyliques ou des halogénures ou des anhydrides d'acides carboxyliques, en les esters carboxiliques correspondants, pour lesquels Z représente

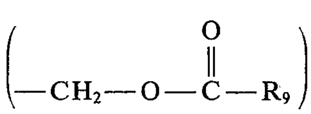

.

ou, par réaction avec des halogénures d'acides sulfoniques, en esters sulfoniques, pour lesquels Z représente $(-CH_2-O-SO_2-R_{10})$,

d) on transforme des composés de formule I dans lesquels Z représente un radical $-COOR_3$ dont le symbole $R_3$ ne représente pas l'hydrogène, de manière connue, en sels (Z = —COO Cat) ou en acides libres (Z = —COOH);

e) on fait réagir des composés de formule I dans lesquels Z représente un radical $-COOR_3$ avec l'ammoniac, des amines ou des hydrazines de manière à obtenir des carboxamides ($Z = -CONHR_5$) ou des carbohydrazides

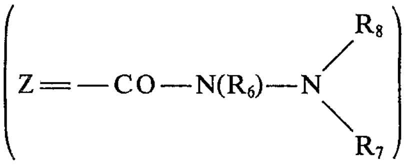

f) on transforme des composés de formule I dans lesquels Z représente un radical —COOH, par réaction avec des halogénures d'acides, puis réaction avec des amines, des hydrazines ou des alcools, respectivement en carboxamides, carbohydrazides ou esters;

g) on transestérifie des composés de formule I dans lesquels Z représente un radical d'ester à bas poids moléculaire, avec des alcools de formule $R_3OH$;

h) on déshydrate des composés de formule I dans lesquels Z représente un radical $-CO-NH_2$ et, si on le désire, on transforme les nitriles obtenus (Z = —CN), par fixation de $H_2S$, en les thiocarboxamides correspondants ($Z = -CS-NH_2$).

3. Produits herbicides caractérisés en ce qu'ils contiennent un composé de formule I.

4. Application de composés de formule I pour la lutte contre des plantes adventices monocotylédones.

5. Composé qui répond à la formule:

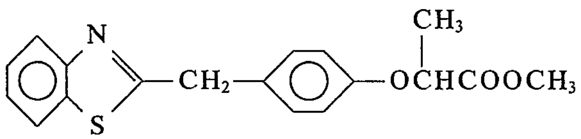

6. Composé qui répond à la formule:

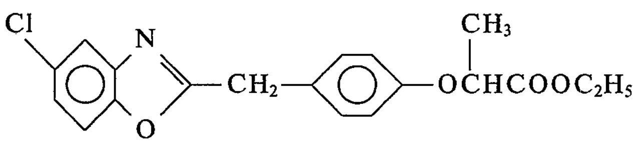

**Revendications pour l'Etat contractant: AT**

1. Produits herbicides caractérisés en ce qu'ils contiennent un composé répondant à la formule I:

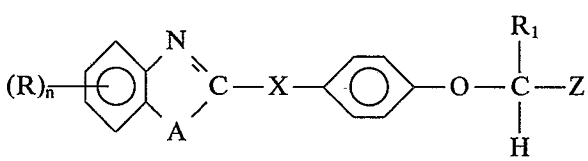

(I)

dans laquelle

R représente un halogène, un radical $CF_3$, $NO_2$, CN, $NH_2$, alkyle en $C_1$—$C_4$, alcoxy en $C_1$—$C_4$, alkylthio en $C_1$—$C_4$, ou un radical phenoxy éventuellement porteur d'un à trois substituants pris dans l'ensemble constitué par les halogènes, $CF_3$, les alkyles en $C_1$—$C_4$, $NO_2$ et les radicaux $-NH-C(O)-R_2$,

n représente un nombre égal à 0, 1, 2, 3 ou 4

A représente O, S, NH ou un radical N-Alkyl en $C_1$—$C_4$,

X représente $-CH_2-$ ou $-CH_2-CH_2-$,

$R_1$ représente H ou un alkyle en $C_1$—$C_4$,

Z représente un radical répondant à l'une des formules suivantes:

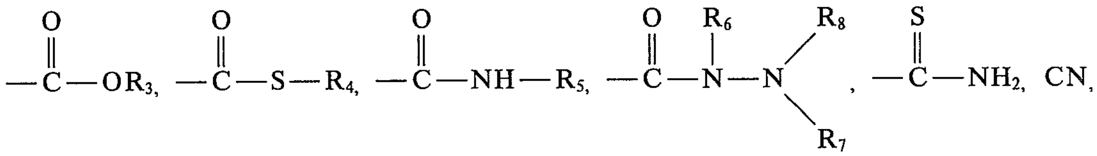

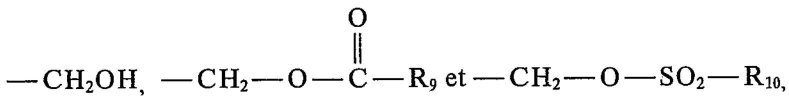

$R_3$ représente:

- un atome d'hydrogéne,
- un alkyle en $C_1$—$C_{12}$ qui porte éventuellement de 1 à 6 atomes d'halogènes, de préférence F, Cl ou Br, et/ou un radical OH, alcoxy en $C_1$—$C_6$, alkylthio en $C_1$—$C_4$, ($C_1$—$C_6$)-alcoxy-($C_2$—$C_6$)-alcoxy, halogéno-alcoxy en $C_1$ ou $C_2$, méthoxy-ethoxy-ethoxy, alkylamino en $C_1$—$C_4$, di-($C_1$—$C_4$)-alkylamino, ($C_1$—$C_4$)-alcoxycarbonyle, phényle, oxirannyle ou phénoxy, ce dernier pouvant lui-même porter un ou deux substituants pris dans l'ensemble constitué par les halogènes et les alkyles en $C_1$—$C_4$,
- un cycloalkyle en $C_5$ ou $C_6$ ou un halogenoalkyle en $C_5$ ou $C_6$,
- un alcényle en $C_3$—$C_6$, un halogénoalcényle en $C_3$—$C_6$, ou un cycloalcényle en $C_5$ ou $C_6$,
- un alcynyle en $C_3$ ou $C_4$, lequel porte éventuellement un ou deux substituants pris dans l'ensemble constitué par les alkyles en $C_1$—$C_6$, le phényle, les halogènes et les alcoxy en $C_1$ et $C_2$,
- un phényle éventuellement porteur d'un à trois substituants pris dans l'ensemble constitué par les alkyles en $C_1$—$C_4$, les alcoxy en $C_1$—$C_4$, les halogènes, $NO_2$ et $CF_3$,
- un furfuryle ou un tétrahydrofurfuryle, ou
- un équivalent cationique d'une base minérale ou organique,

$R_4$ représente:

- un alkyle en $C_1$—$C_6$, éventuellement porteur d'un alcoxy en $C_1$—$C_4$, d'un halogène ou d'un phényle, ce dernier pouvant lui-même porter de un à trois substituants pris dans l'ensemble constitué par les alkyles en $C_1$—$C_4$ et les halogènes,
- un alcényle en $C_3$—$C_6$, ou
- un phényle éventuellement porteur d'un à trois substituants pris dans l'ensemble constitué par les alkyles en $C_1$—$C_4$ t les halogènes,

$R_5$ représente:
H, un alkylène en $C_1$—$C_6$, un hydroxylalkyle en ($C_1$—$C_6$) ou un cycloalkyle en $C_5$ ou $C_6$,

$R_6$ représente:
H ou $CH_3$,

$R_7$ représente:
H, $CH_3$ ou $C_2H_5$,

$R_8$ représente:
H, $CH_3$, $C_2H_5$ ou un phényle,

$R_9$ représente:
un alkyle en $C_1$—$C_6$, éventuellement porteur d'un à trois atomes d'halogènes, un cycloalkyle en $C_3$, $C_5$ ou $C_6$, un alcényle en $C_3$—$C_6$, un phényle, un alkylphényle à alkyle en $C_1$—$C_4$, un alcoxyphényle à alcoxy en ($C_1$—$C_4$), un halogènophényle, un trifluorméthyl-phényle ou un nitrophényle,

$R_{10}$ représente:
un alkyle en $C_1$—$C_4$ ou un phényle, ce dernier pouvant également porter de un à trois substituants pris dans l'ensemble constitué par les halogènes, $CF_3$, $NO_2$ et les alkyles en $C_1$—$C_4$.

2. Application de composés de formule I à la lutte contre des plantes adventices monocotylédones.